(19) Europäisches Patentamt / European Patent Office / Office européen des brevets

(11) **EP 3 160 506 B1**

## (12) FASCICULE DE BREVET EUROPEEN

(45) Date de publication et mention de la délivrance du brevet:
**28.08.2024   Bulletin 2024/35**

(21) Numéro de dépôt: **15731623.3**

(22) Date de dépôt: **30.06.2015**

(51) Classification Internationale des Brevets (IPC):
**A61K 45/06** (2006.01)    **A61K 31/215** (2006.01)
**A61K 31/4402** (2006.01)    **A61K 31/341** (2006.01)
**A61K 36/53** (2006.01)    **A61P 33/00** (2006.01)
**A61P 33/14** (2006.01)    **A01N 51/00** (2006.01)
**A61K 8/92** (2006.01)    **A61K 9/00** (2006.01)
**A61K 47/00** (2006.01)

(52) Classification Coopérative des Brevets (CPC):
(C-Sets disponibles)
**A61K 36/53; A01N 43/40; A01N 47/02;
A01N 49/00; A01N 51/00; A01N 53/00;
A61K 8/922; A61K 9/0017; A61K 31/215;
A61K 31/341; A61K 31/4402; A61K 45/06;
A61P 33/00; A61P 33/14; A61Q 17/02**       (Cont.)

(86) Numéro de dépôt international:
**PCT/EP2015/064832**

(87) Numéro de publication internationale:
**WO 2016/001216 (07.01.2016 Gazette 2016/01)**

## (54) NOUVEAU PROCEDE D'APPLICATION TOPIQUE D'AGENTS VETERINAIRES

NEUES VERFAHREN DER TOPISCHEN ANWENDUNG VON VETERINÄRSUBSTANZEN

NOVEL METHOD FOR TOPICAL APPLICATION OF VETERINARY AGENTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **30.06.2014   EP 14306049**

(43) Date de publication de la demande:
**03.05.2017   Bulletin 2017/18**

(73) Titulaire: **Ceva Santé Animale
33500 Libourne (FR)**

(72) Inventeurs:
- **GUIMBERTEAU, Florence
  F-33450 Montussan (FR)**
- **KAREMBE, Hamadi
  F-33500 Libourne (FR)**
- **KALTSATOS, Vassilios
  F-33500 Libourne (FR)**
- **BARTHELEMY, Gautier
  F-33800 Bordeaux (FR)**

(74) Mandataire: **Cabinet Becker et Associés
25, rue Louis le Grand
75002 Paris (FR)**

(56) Documents cités:
WO-A1-2011/120427       WO-A1-2012/107585
JP-A- 2002 193 755       US-A- 6 010 492
US-A1- 2010 137 451

- **"VECTRA-3D ANNEXE I RÉSUMÉ DES CARACTÉRISTIQUES DU PRODUIT", 26 March 2014 (2014-03-26), XP055157821, Retrieved from the Internet <URL:http://www.ema.europa.eu/docs/fr_FR/document_library/EPAR_-_Product_Information/veterinary/002555/WC500163785.pdf> [retrieved on 20141210]**

- **"GAMME ANTIPARASITAIRE EXTERNE SERESTO LEAFLET", 9 December 2013 (2013-12-09), XP055157759, Retrieved from the Internet <URL:http://notices.plantes-et-jardins.com/Bayer/leaflet seresto.pdf> [retrieved on 20141210]**
- **"FRONTLINE leaflet", 3 August 2011 (2011-08-03), XP055157792, Retrieved from the Internet <URL:http://www.lloydspharmacy.com/wcsstore7.00.00.223/ExtendedSitesCatalogAssetStore/images/pil/3266889.pdf> [retrieved on 20141210]**
- **ANONYMOUS: "Virbac Animal Health - PYRETHRIN DIP(TM) Anti-Parasitic | United States", 21 November 2013 (2013-11-21), XP055157799, Retrieved from the Internet <URL:http://web.archive.org/web/20131121014603/http://www.virbacvet.com/products/detail/pyrethrin-dip/anti-parasitic/external-parasitics> [retrieved on 20141210]**

(52) Classification Coopérative des Brevets (CPC):
(Cont.)

C-Sets
**A01N 43/40, A01N 25/02, A01N 53/00;
A01N 47/02, A01N 25/02, A01N 49/00;
A01N 49/00, A01N 25/02;
A01N 51/00, A01N 25/02, A01N 43/40,
A01N 53/00;
A01N 53/00, A01N 25/02;
A61K 31/215, A61K 2300/00;
A61K 31/341, A61K 2300/00;
A61K 31/4402, A61K 2300/00;
A61K 36/53, A61K 2300/00**

**Description**

La présente invention concerne des méthodes, appareils et compositions pour l'application topique d'agents vétérinaires. Elle concerne plus spécifiquement des méthodes, appareils et compositions permettant l'application topique et séquentielle de composés vétérinaires pour assurer une meilleure observance des traitements.

ARRIERE-PLAN DE L'INVENTION

Les traitements vétérinaires se présentent sous différentes formes et principalement sous forme injectable, de shampoings, de sprays, ou de dépôt cutané.

Les traitements par shampoings exigent une application sur toute la surface de la peau de l'animal et une étape de rinçage à l'eau, souvent désagréable pour celui-ci. En outre, les traitements utilisant des shampoings ne peuvent s'appliquer à tous les agents thérapeutiques et ne fournissent qu'un traitement de courte durée et transitoire.

L'application de traitement en spray peut se révéler assez compliquée, de nombreux animaux ne supportant ni le bruit ni l'odeur du spray. De plus, il est nécessaire de compter le nombre de pressions pour adapter la posologie au poids de l'animal traité et veiller à appliquer uniformément le produit sur l'ensemble du corps. Il est aussi nécessaire d'appliquer le traitement à 48 heures de distance de tout bain. Enfin, une fois l'animal traité par spray, le pelage de ce dernier prend souvent une apparence détrempée et poisseuse.

Une voie alternative d'application basée sur le dépôt cutané (en anglais "spot on" ou "pour on") est avantageuse par son efficacité, sa rapidité d'action, ainsi que par l'aspect agréable du poil des animaux après application et séchage. Ce type d'application « spot-on » consiste à appliquer sur une peau sèche, tout en écartant les poils pour rendre bien visible la peau, une composition sous forme de gouttes sur une zone de surface inférieure à 10 cm$^2$, notamment comprise entre 5 et 10 cm$^2$. Après dépôt/libération, la composition ou le composé diffuse puis sèche. Cependant, l'application de type « spot-on » ou « pour-on » présente certains inconvénients. En particulier, il existe une certaine imprécision sur les doses administrées car le produit peut facilement se perdre en coulant sur le poil. Il est donc nécessaire d'effectuer un ou plusieurs dépôts sur le dos de l'animal pour être certain d'administrer une dose efficace. L'importante quantité de produit déposée en un ou plusieurs points de l'animal peut par ailleurs induire des réactions de type inflammatoire (érythème ou prurit par exemple). En outre, le fait de déposer une grande quantité de produit sur un ou plusieurs points se traduit souvent par la présence de produit non administré sur les poils de l'animal, ce qui peut engendrer également des risques pour la santé et la sécurité de l'utilisateur.

Ainsi, il persiste aujourd'hui, dans le domaine de la santé animale, un besoin de nouvelles stratégies d'administration de composés vétérinaires permettant d'améliorer les traitements tout en maintenant, pour l'utilisateur, une sécurité maximale.

Les inventeurs ont mis au point une nouvelle approche pour le traitement des animaux, qui consiste à appliquer de manière topique et séquentielle une composition vétérinaire. Les inventeurs ont montré que cette application topique séquentielle présente des avantages, notamment pour les traitements contre les parasites, par rapport à l'application « spot-on » ou « pour-on », tels que la prévention des effets secondaires et l'efficacité.

RESUME DE L'INVENTION

Les inventeurs ont mis au point une nouvelle méthode pour le traitement des animaux qui consiste à administrer une composition ou un composé vétérinaire de manière topique et séquentielle selon un mode d'application déterminé. Les inventeurs ont montré que cette méthode permettait un meilleur contrôle des doses et une plus grande efficacité d'action, sans effet secondaire pour les animaux. En particulier, les inventeurs ont montré, de manière surprenante, que l'administration séquentielle selon les revendications présentes, avec une dose initiale (dite de charge) de composés vétérinaires pouvait permettre d'obtenir, à doses totales égales, un effet supérieur à celui obtenu notamment par application « spot-on » ou « pour-on ».

Un autre objet de l'invention concerne un composé pour son utilisation pour le traitement thérapeutique selon la revendication 1.

Un autre objet de l'invention concerne l'utilisation d'un composé pour la fabrication d'une composition destinée au traitement thérapeutique ou cosmétique d'un mammifère non humain par application topique séquentielle d'une dose totale déterminée dudit composé, caractérisée en ce que l'application est réalisée selon une posologie comprenant (i) une dose initiale D0, appliquée en début de traitement, représentant au maximum 65% de la dose totale déterminée, et (ii) une pluralité de doses d'entretien Di, appliquées de manière séquentielle pendant la durée du traitement. La somme des doses Di et de la dose D0 est égale à la dose totale déterminée.

Le composé (ou une composition comprenant le composé) peut être appliqué sur la peau ou le poil du mammifère non humain de manière manuelle (pipette, spray) ou automatisée.

Un autre objet de l'invention concerne un dispositif pour la délivrance d'un composé selon la revendication 1 à un chien, caractérisé en ce qu'il comprend une dose totale déterminée dudit composé et un système de libération séquentielle contrôlée assurant la délivrance d'une dose initiale D0 du composé en début de traitement, ladite dose D0 représentant 0,5 à 65% de la dose totale déterminée, et d'une pluralité de doses d'entretien Di du composé, la somme des doses Di et de la dose D0 étant égale à la dose totale déterminée. Le dispositif peut être par exemple une pipette ou un collier, notamment un collier comprenant une buse de dispersion, etc.

L'invention est applicable à tout composé (ou mélange de composés) vétérinaire, et notamment des composés antiparasitaires, cosmétiques ou dermatologiques. Elle peut être mise en oeuvre chez tout mammifère non-humain, comme notamment des animaux de compagnie (canins, félins, etc.), des animaux d'élevage (bovins, ovins, porcins, etc.), des chevaux, etc.

LEGENDE DES FIGURES

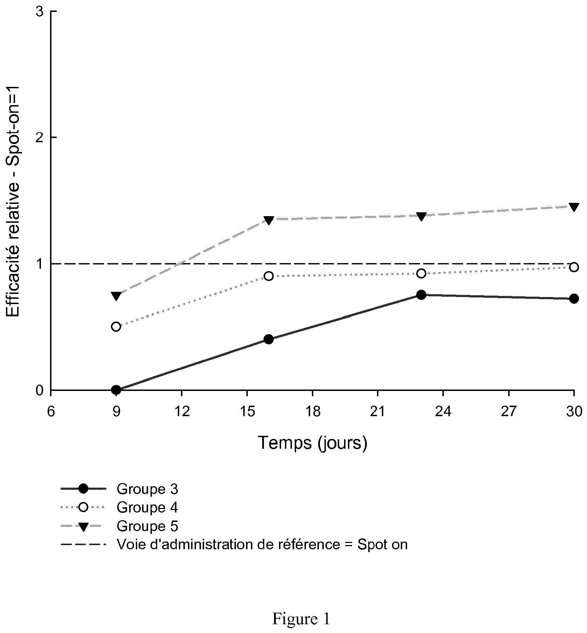

Figure 1 : Efficacité comparée d'un antiparasitaire appliqué par voie topique par la technique du « spot-on » et par la méthode selon l'invention.

DESCRIPTION DETAILLEE DE L'INVENTION

La présente invention est relative des compositions, et appareils/dispositifs pour le traitement des chiens. Elle concerne plus particulièrement des méthodes d'administration ou de traitement basées sur l'application topique et séquentielle, selon un mode d'application déterminé, d'un composé à usage vétérinaire, ainsi que des compositions et appareils adaptés à sa mise en oeuvre. Les inventeurs ont montré que cette méthode d'administration permettait une meilleure observance du traitement, sans effet secondaire pour les animaux.

Un objet de l'invention concerne donc un composé (ou une composition) pour son utilisation pour le traitement d'un chien par application topique séquentielle d'une dose totale déterminée dudit composé, caractérisé en ce que l'application est réalisée selon une posologie comprenant (i) une dose initiale D0, appliquée en début de traitement, représentant au maximum 65% de la dose totale déterminée, et (ii) une pluralité de doses d'entretien Di, appliquées de manière séquentielle pendant la durée du traitement, la somme des doses Di et de la dose D0 étant égale à la dose totale déterminée.

Protocole

Comme indiqué, l'invention repose notamment sur la démonstration surprenante que la répartition de la quantité totale efficace d'un composé à usage vétérinaire en plusieurs doses (ou fractions) séquentielles, dont une dose initiale (de charge D0) et des doses d'entretien (Di ou di), permet d'obtenir un meilleur effet que l'application topique de la dose totale déterminée (D).

Ainsi, pour une dose totale déterminée, le protocole utilise des fractions administrées séquentiellement selon la formule suivante ;

$$D = D0 + \sum_{i=0}^{n} di$$

dans laquelle :

- D : Dose totale déterminée,
- D0 : Dose initiale (dose de charge),
- $d_i$ ou Di : Doses d'entretien, et
- i le nombre d'application séquentielle de la dose d'entretien.

Pour un composé/une composition donné(e), la dose totale déterminée correspond typiquement à la dose efficace recommandée par le praticien, qui est généralement connue dans la littérature et/ou déterminée par l'homme du métier sur la base de ses connaissances générales ou par la réalisation de tests préliminaires ou cliniques. Par exemple, pour un antiparasitaire agissant contre les puces et les tiques comme le Frontline® Combo (mélange de fipronil et S-methoprène commercialisé par la société Merial), la dose totale déterminée sera typiquement de 5 mg de fipronil et de 6 mg de S-méthoprène par kg de poids corporel pour un chat, et de 6,7 mg de fipronil et de 6 mg de S-méthoprène par kg de poids corporel pour un chien. La dose totale déterminée peut ainsi varier selon le mammifère non humain considéré, son poids et bien entendu la nature du composé à usage vétérinaire. Pour le traitement selon l'invention, la dose totale déterminée est répartie en plusieurs fractions qui seront administrées de manière séquentielle, tout d'abord une dose initiale dite de charge (D0) puis une pluralité de doses d'entretien Di.

La dose initiale (D0) pourra être adaptée par l'homme du métier en fonction de la classe ou de la nature du composé/de la composition appliquée, du mammifère non humain cible et de la durée du traitement.

Néanmoins, dans un mode préféré de mise en oeuvre, la dose initiale D0 représente de 0,5% à 60% de la dose totale déterminée, de préférence de 1% à 60%. Plus préférentiellement, lorsque la durée du traitement visée est inférieure à 2 mois, la dose initiale D0 représente avantageusement de 10-60% de la dose totale déterminée, en particulier de 15-60%, de 20-60%, de 20 à 50% ou de 30 à 50%. Lorsque la durée du traitement visée est supérieure à 2 mois, la dose initiale D0 représente de préférence de 0,5 à 10%, de 0,5 à 8%, plus préférentiellement de 1 à 5%, de la dose totale déterminée.

Comme le montrent les résultats présentés sur la figure 1, pour une composition antiparasitaire comme le Vectra3D (Ceva Santé Animale), des doses de charge de 30% ou 50% donnent un effet supérieur au traitement topique de type « spot- on » de référence alors que, sans dose de charge, les résultats restent inférieurs. En outre avec une dose de charge représentant 20% de la dose totale, les résultats d'efficacité observés montrent une augmentation continue de l'efficacité antipuces dans le temps, ce qui se traduit par une efficacité du traitement accrue par rapport à une application topique de type « spot-on ».

Le composé est un agent thérapeutique selon la revendication 1, avec une durée de traitement visée inférieure à 2 mois, la dose initiale D0 représente avantageusement de 10-60% de la dose totale déterminée, de préférence de 15-60%, plus préférentiellement de 20 à 60%, de 20 à 50% ou encore plus préférentiellement de 30-50%

Après application ou libération de la dose initiale, le traitement de l'invention comprend l'application ou la libération de plusieurs doses d'entretien (Di). Dans le cadre de l'invention, les doses d'entretien peuvent être identiques ou variables au cours de traitement. De manière préférée néanmoins, toutes les doses d'entretien Di sont identiques.

De préférence, chaque dose d'entretien Di représente au plus 35% de la dose initiale D0, plus préférentiellement au plus 20%, encore plus préférentiellement au plus 10%. Par ailleurs, la fréquence d'application des doses d'entretien Di peut être constante tout au long du traitement, ou variable.

Dans un mode préféré, les doses d'entretien Di sont identiques et leur fréquence d'application est constante au cours du traitement. Avantageusement, la fréquence d'application des doses d'entretien Di est comprise entre 1h et 1 mois, de préférence entre 2h et 15 jours, plus préférentiellement entre 4h et 7 jours, encore plus préférentiellement entre 12h et 4 jours.

La durée du traitement, ou la fréquence d'application et/ou la quantité des doses d'entretien Di peuvent être ajustées par l'homme du métier en fonction du composé, du mammifère non humain et du type de traitement.

## Administration topique

L'invention comprend l'administration topique séquentielle d'un ou plusieurs composés, selon un mode d'application déterminé. L'administration est topique et préférentiellement localisée sur la peau, en surface.

Dans un mode préféré de l'invention, le composé ou la composition est administré sur une zone de la peau de l'animal, par exemple le cou ou toute zone pratique (par exemple le dos, entre les épaules, etc.) au moyen d'un dispositif adapté à la zone visée comme par exemple un collier, un spray ou une buse de dispersion. L'administration peut être réalisée au moyen de tout dispositif comprenant un réservoir contenant le composé.

Dans un mode préféré, le composé ou la composition est administré au moyen d'un dispositif à libération contrôlée, par exemple un collier. D'autres dispositifs modes à libération contrôles sont mentionnés par exemple dans les brevets US7,140,325 ou US6,010,492.

L'administration du composé ou de la composition est poursuivie généralement jusqu'à ce que l'intégralité de la dose totale déterminée soit administrée. Bien entendu, le traitement peut être interrompu si le but est atteint avant ou si le praticien le décide.

## Composé

Le composé est un agent thérapeutique qui comprend un néonicotinoïde, de préférence le dinotefuran, une pyréthrinoïde, de préférence la perméthrine ou la fluméthrine et un IGR, de préférence le pyriproxyfène.

Les différentes classes de parasites et classes d'agents antiparasitaires ainsi que les agents actifs commercialisés peuvent être retrouvés sur le site internet parasitipedia à l'adresse suivante : http://parasitipedia.net/; et sur le site internet European Scientific Counsel Companion Animal Parasite (ESCCA) à l'adresse suivante : http://www.esccap.org/

Par «agent cosmétique ou dermatologique » on entend une substance ou un mélange en vue, exclusivement ou principalement, de nettoyer, de parfumer, de modifier l'aspect, de protéger, de traiter, prévenir ou maintenir en bon état les parties superficielles (épiderme, systèmes pileux...) ou corriger les odeurs corporelles de mammifères non-humains. Les agents cosmétiques ou dermatologiques selon l'invention peuvent être d'origine naturelle ou synthétique ou une combinaison des deux.

A titre d'exemple, on peut citer des agents hydratants, des émollients, des parfums, des extraits de plantes, des extraits d'algues, des vitamines, des huiles essentielles ou des antibiotiques.

## Composition

Par « composition » on entend toute formulation mise en oeuvre pour appliquer un composé, qu'il soit pur ou sous forme de mélange, comprenant en outre un véhicule ou excipient vétérinairement acceptable.

Par excipient vétérinairement acceptable, on entend un excipient toléré par le mammifère non humain lorsqu'il est appliqué topiquement, et qui est capable de dissoudre suffisamment et/ou formuler le composé.

La composition est préférentiellement une forme liquide comprenant de préférence un ou des solvants organiques comme notamment un alcool alkylique en C1-C4, tels que l'éthanol, l'isopropanol, ou le méthanol ; l'acétyltributylcitrate ; un ou des esters d'acides gras tels que l'ester de diméthyle et l'adipate de diisobutyle ; l'acétone, l'acétonitrile, l'alcool benzylique, le butyldiglycol, le diméthylacétamide, le diméthylformamide, le dipropylène glycol n-butyl éther, l'éthylène glycol monoéthyl éther, l'éthylène glycol éther monométhylique, le diéthylène glycol monoéthyl éther, le monomethylacetamide, le monométhyléther de dipropylèneglycol, les polyoxyéthylène-glycols liquides, le propylèneglycol, les 2-pyrrolidones comme la N-méthyl-pyrrolidone et le N-Octyl-2-pyrrolidone, le propylène carbonate, le diéthylène glycol monoéthyl éther, le dimethyl sulfoxide, l'éthylène glycol, le phtalate de diéthyle, l'éthoxydiglycol, ou leurs combinaisons.

Dans un mode de réalisation, le solvant organique comprend du N-méthyl-pyrrolidone et du N-Octyl-2-pyrrolidone. Dans un mode de réalisation préférentiel, le solvant organique comprend de l'isopropanol, du myristate d'isopropyl et des triglycérides à chaines moyennes seul ou combiné. Dans un mode de réalisation encore plus préférentiel, le solvant organique comprend du dimethyl sulfoxide.

La composition de la présente invention peut également comprendre un ou des agents ou adjuvants supplémentaires tels qu'un ou des co-solvants, colorants, agents d'étalement, antioxydants, stabilisants à la lumière et/ou agents d'adhésivité.

Mammifères non humain

L'invention est adaptée au traitement de chiens D'autres aspects et avantages de l'invention apparaîtront à la lecture des exemples qui suivent, qui doivent être considérés comme illustratifs et non limitatifs.

EXEMPLES

Exemple 1 : Étude d'efficacité d'un traitement d'un mois avec un antiparasitaire sur les tiques

Quarante (40) chiens de 10 à 25kg ont été inclus dans l'étude et séparés en cinq groupes :

- Groupe 1 : groupe témoin constitué de 8 chiens non traités
- Groupe 2 : Groupe « Spot -on » ou groupe « voie d'administration de référence : spot-on » est constitué de 8 chiens. Sur chaque chien est administré par « spot on » 3,6mL (= D : Dose totale déterminée) de Vectra 3D (Ceva Santé Animale) en trois points (entre les omoplates, au milieu du dos et à la base de la queue) au jour J0.
- Groupe 3 : ce groupe est constitué de 8 chiens. Sur chaque chien est administré un volume total de 3,6mL (100% de D) de Vectra3D continue de J0 à J28 à raison d'environ 0,124mL par jour.
- Groupe 4 : ce groupe est constitué de 8 chiens. Sur chaque chien est administrée à J0 une dose initiale de 0,72mL (20% de D) de Vectra 3D puis 2,88mL de façon continue en doses quotidiennes identiques de J1 à J28 (0,10mL par jour).
- Groupe 5 : ce groupe est constitué de 8 chiens. Sur chaque chien est administrée à J0 une dose initiale de 1,08 mL (30% de D) de Vectra 3D puis 2.52mL de façon continue en doses quotidiennes identiques de J1 à J28 (0,09mL par jour).

Tous les 40 chiens de cette étude sont infestés par 50 ($\pm$ 4) tiques *Dermacentor reticulatus* adulte (*D. reticulatus*) à J-7 puis à J-2, J7, J14, J21 et J28. Les tiques sont comptées et enlevées 2 jours ($\pm$ 2 h) après l'infestation soit à J-5, J0, J9, J16, J23 et J30. Pour chaque chien, le nombre de tiques mortes à chaque point (J-5, J0, J9, J16, J23 et J30) est déterminé. Il est égal au nombre de tique implantées (50) moins le nombre de tiques vivantes (gorgées de sang) comptées et enlevées aux points d'observation. Un nombre moyen de tiques mortes est alors calculé en faisant la moyenne arithmétique pour chaque groupe.

L'efficacité relative est alors calculée comme le rapport entre le nombre de tiques tuées dans les groupes traités 2, 3, 4 et 5 par rapport au nombre de tiques comptées dans le groupe témoin. Les résultats d'efficacité pour les groupes 2, 3, 4, et 5 sont présentés dans la figure 1.

Durant cette étude, aucun des chiens présents dans les groupes traités (groupes 2, 3, 4, et 5) n'a présenté de signe d'inflammation au niveau des points d'applications. De plus aucun signe clinique lié aux traitements n'a été observé durant cet essai.

Résultats :

Avec une dose initiale D0 de 30% suivie de doses d'entretien journalières identiques, une différence significative est obtenue par rapport à une application « Spot on ». On peut conclure que l'effet antiparasitaire produit par le mode d'administration selon la présente invention est plus soutenu que celui obtenu par une application de type « spot-on ». Ces résultats illustrent l'efficacité et le caractère inattendu de la méthode de l'invention qui, à dose totale égale, démontre un effet antiparasitaire supérieur.

Exemple 2 : Étude d'efficacité pour une durée de traitement supérieure à 2 mois avec une préparation dermocosmétique

Un mélange d'huiles essentielles (0,5% romarin et 0,5% lavande) dispersé dans de l'isopropyl myristate est préparé. Une dose totale déterminée de 31 mL de ce mélange est appliquée sur un groupe de 8 chiens selon le protocole suivant :

Application d'une dose initiale de 1 mL de mélange d'huiles essentielles à la base du cou, puis une dose d'entretien de 0.25mL (0,8%) à la base du cou tous les trois jours pendant 360 jours;
Le groupe témoin est composé de 8 chiens n'ayant reçu aucun traitement.

L'aspect du poil (brillance et touché soyeux) des animaux traités est sensiblement amélioré à l'issu du traitement.

**Revendications**

1. Composé, lequel étant un agent thérapeutique comprenant du dinotéfurane, de la perméthrine, et du pyriproxyfène, pour son utilisation pour le traitement d'un chien par administration sur la peau d'une dose totale déterminée dudit composé, **caractérisé en ce**

**que** l'application est réalisée selon une posologie comprenant (i) une dose initiale D0, appliquée en début de traitement, représentant 30 à 50%, de la dose totale déterminée, et (ii) une pluralité de doses d'entretien Di, appliquées de manière séquentielle pendant la durée du traitement, la somme des doses Di et de la dose D0 étant égale à la dose totale déterminée, et chaque dose d'entretien Di représente au plus 10 % de la dose initiale D0, avec une durée du traitement visée inférieure à 2 mois.

2. Composé pour son utilisation selon la revendication 1, **caractérisé en ce que** toutes les doses d'entretien Di sont identiques.

3. Composé pour son utilisation selon l'une quelconque des revendications précédentes, **caractérisé en ce que** la fréquence d'application des doses d'entretien Di est constante tout au long du traitement.

4. Composé pour son utilisation selon l'une quelconque des revendications précédentes, **caractérisé en ce que** le composé est formulé dans une composition avec au moins un véhicule ou excipient acceptable.

5. Composé pour son utilisation selon l'une quelconque des revendications précédentes, **caractérisé en ce que** le composé est appliqué au moyen d'un dispositif à libération contrôlée, de préférence un collier.

6. Dispositif pour la délivrance d'un composé, lequel étant un agent thérapeutique comprenant du dinotéfurane, de la perméthrine, et du pyriproxyfène, à un chien, **caractérisé en ce qu'**il comprend une dose totale déterminée dudit composé et un système de libération séquentielle contrôlée assurant la délivrance d'une dose initiale D0 du composé en début de traitement, ladite dose D0 représentant 30 à 50% de la dose totale déterminée, et d'une pluralité de doses d'entretien Di du composé, la somme des doses Di et de la dose D0 étant égale à la dose totale déterminée, et chaque dose d'entretien Di représente au plus 10 % de la dose initiale D0, avec une durée du traitement inférieure à 2 mois.

**Patentansprüche**

1. Verbindung, die ein therapeutisches Mittel ist, umfassend Dinotefuran, Permethrin und Pyriproxyfen, zur Verwendung bei der Behandlung eines Hundes durch Verabreichen einer bestimmten Gesamtdosis der Verbindung auf die Haut, **dadurch gekennzeichnet, dass** die Anwendung gemäß einer Dosierung erfolgt, umfassend (i) eine Anfangsdosis D0, die zu Beginn der Behandlung angewendet wird, die 30 bis 50 % der bestimmten Gesamtdosis ausmacht, und (ii) eine Vielzahl von Erhaltungsdosen Di, die während der Dauer der Behandlung auf sequenzielle Weise angewendet werden, wobei die Summe der Dosen Di und der Dosis D0 gleich der bestimmten Gesamtdosis ist und jede Erhaltungsdosis Di höchstens 10 % der Anfangsdosis D0 ausmacht, mit einer angestrebten Behandlungsdauer von weniger als 2 Monaten.

2. Verbindung zur Verwendung nach Anspruch 1, **dadurch gekennzeichnet, dass** alle Erhaltungsdosen Di identisch sind.

3. Verbindung zur Verwendung nach einem der vorstehenden Ansprüche, **dadurch gekennzeichnet, dass** die Anwendungshäufigkeit der Erhaltungsdosen Di während der ganzen Behandlung konstant ist.

4. Verbindung zur Verwendung nach einem der vorstehenden Ansprüche, **dadurch gekennzeichnet, dass** die Verbindung in einer Zusammensetzung mit mindestens einem akzeptablen Träger oder Hilfsstoff formuliert ist.

5. Verbindung zur Verwendung nach einem der vorstehenden Ansprüche, **dadurch gekennzeichnet, dass** die Verbindung mittels einer Vorrichtung für eine kontrollierte Freisetzung, vorzugsweise eines Halsbands, angewendet wird.

6. Vorrichtung für die Abgabe einer Verbindung, die ein therapeutisches Mittel ist, umfassend Dinotefuran, Permethrin und Pyriproxyfen, an einen Hund, **dadurch gekennzeichnet, dass** sie eine bestimmte Gesamtdosis der Verbindung und ein System für die kontrollierte sequenzielle Freisetzung umfasst, das die Abgabe einer Anfangsdosis D0 der Verbindung zu Beginn der Behandlung, wobei die Dosis D0 30 bis 50 % der bestimmten Gesamtdosis ausmacht, und einer Vielzahl von Erhaltungsdosen Di der Verbindung sicherstellt, wobei die Summe der Dosen Di und der Dosis D0 gleich der bestimmten Gesamtdosis ist und jede Erhaltungsdosis Di höchstens 10 % der Anfangsdosis D0 ausmacht, mit einer Behandlungsdauer von weniger als 2 Monaten.

**Claims**

1. A compound, being a therapeutic agent comprising dinotefuran, permethrin and pyriproxyfen, for use in the treatment of a dog by administering a determined total dose of said compound to the skin, **characterized in that** the application is carried out according to a dosage regimen comprising (i) an initial dose D0, applied at the beginning of treatment, representing 30 to 50% of the determined total dose, and (ii) a plurality of maintenance doses Di applied sequentially for the duration of the treatment, the sum of the doses Di and the dose D0 being equal to the determined total dose, and each maintenance dose Di representing at most 10% of the initial dose D0, with a targeted treatment duration of less than 2 months.

2. The compound for use according to claim 1, **characterized in that** all the maintenance doses Di are identical.

3. The compound for use according to any of the preceding claims, **characterized in that** the frequency of application of the maintenance doses Di is constant throughout the treatment.

4. The compound for use according to any of the preceding claims, **characterized in that** the compound is formulated in a composition with at least one acceptable excipient or carrier.

5. The compound for use according to any of the preceding claims, **characterized in that** the compound is applied by means of a controlled-release device, preferably a collar.

6. A device for delivering a compound to a dog, the compound being a therapeutic agent comprising dinotefuran, permethrin and pyriproxyfen, **characterized in that** it comprises a determined total dose of said compound and a controlled sequential release system ensuring the delivery of an initial dose D0 of the compound at the beginning of treatment, said dose D0 representing 30 to 50% of the determined total dose, and of a plurality of maintenance doses Di of the compound, the sum of the doses Di and the dose D0 being equal to the determined total dose, and each maintenance dose Di representing at most 10% of the initial dose D0, with a treatment duration of less than 2 months.

Figure 1

**EP 3 160 506 B1**

**RÉFÉRENCES CITÉES DANS LA DESCRIPTION**

**Documents brevets cités dans la description**

- US 7140325 B
- US 6010492 A